# EUROPEAN PATENT SPECIFICATION

(11) **EP 0 839 800 B1**
(45) Date of publication and mention of the grant of the patent: **23.10.2002**
(21) Application number: 97120490.4
(22) Date of filing: 18.06.1993
(51) Int. Cl.: C07C 231/02, C07C 233/07, C07D 207/27

(54) **Process for preparing halogenoacetamide derivatives**
Verfahren zur Herstellung von Halogenacetamidderivaten
Procédé de préparation de dérivés d'halogénoacétamide

(30) Priority: 19.06.1992 JP 16049692
(43) Date of publication of application: 06.05.1998
(62) Divisional of application: 93109812.3
(73) Proprietor: DAIICHI PHARMACEUTICAL CO., LTD., Chuo-ku, Tokyo 103 (JP)
(72) Inventor: Kamihara, Shinji, c/o Daiichi Pharm. Co., Ltd., Edogawa-ku, Tokyo (JP); Kaneuchi, Tohru, c/o Daiichi Pharm. Co., Ltd., Edogawa-ku, Tokyo (JP); Uchiyama, Keiji, c/o Daiichi Pharm. Co., Ltd., Edogawa-ku, Tokyo (JP); Terada, Tatsuya, c/o Daiichi Pharm. Co., Ltd., Edogawa-ku, Tokyo (JP)
(74) Representative: Kinzebach, Werner, Dr.

(56) References cited:
- EP-A- 0 008 057
- EP-A- 0 065 804
- CH-A- 518 266
- FR-A- 2 216 267
- GB-A- 1 039 113
- GB-A- 2 053 909
- US-A- 2 863 752
- US-A- 3 647 876
- US-A- 3 867 446

## Description

The present invention relates to a process for preparing halogenoacetamide derivatives and based thereon, to a process for preparing pyrrolidinylacetamide derivatives using said halogenoacetamide derivatives as intermediates. The pyrrolidinylacetamide derivatives are useful as an agent for improving cerebral functions.

### BACKGROUND OF THE INVENTION

Known processes for preparing pyrrolidinylacetamide derivatives include a process comprising reacting pyrrolidinylacetic acid or a reactive derivative thereof with an amine in an organic solvent in the presence of dicyclohexylcarbodiimide as disclosed in U.S. Patent 4,341,790 and JP-A-56-2960 (the term "JP-A" as used herein means an "unexamined published Japanese patent application").

The above process is not suited for production on an industrial- scale due to disadvantages, such as use of expensive dicyclohexylcarbodiimide as condensing agent, involvement of complicated steps for separation and removal of dicyclohexylurea which is a by-product from dicyclohexylcarbodiimide, and poor overall yield. Moreover, the starting material, e.g., 2-oxo-1-pyrrolidinylacetic acid, should be produced by reacting 2-pyrrolidinone with methyl bromoacetate and hydrolyzing the resulting methyl 2-oxo-1-pyrrolidinylacetate, and purified by distilling the reaction mixture under high pressure and at high temperature. These complicated operations and low yields are disadvantageous for industrial production.

The preparation of N-substituted 2-chloroacetamides is described in various documents. For instance US 3,867,446 relates to the synthesis of N-alkyl-substituted chloroacetanilides, e.g. N-isopropyl-chloroacetanilide, by reacting the appropriate N-alkyl-anilide with chloroacetic acid in the presence of phosphorous oxychloride at a temperature above 150 °C under anhydrous conditions. Similarly, US 2,863,752 and US 3,647,876 describe processes wherein secondary amines are reacted with chloroacetyl chloride in a system consisting of an organic solvent and aqueous caustic soda (sodium hydroxide). According to FR 2,216,267 A a secondary amine is reacted with chloroacetyl chloride in an organic solvent and an aqueous solution of sodium hydroxide is added after the reaction is completed.

While the above-mentioned documents relate to processes wherein chloroacetyl chloride is reacted with secondary amines, its reaction with primary amines is described, for instance, in EP 0008057 A and CH 518266 A.

According to CH 518266 A 2,6-xylidine is reacted with chloroacetyl chloride in an aqueous solution. Water and potassium hydroxide are used. It is required to filter the reaction mixture to obtain a crude product which has to be recristallized from a mixture of water and ethanol.

According to EP 0008057 A appropriate amines are reacted with chloroacetyl chloride in a 1-phase system. Toluene and potassium carbonate are used. It is required to filter the reaction mixture in order to remove by-products which are formed during the reaction.

On the other hand, a process for preparing N-substituted lactams is known as desclosed in British Patent No. 1,039,113. This known process comprises reacting a N-unsubstituted lactam with an alkali metal hydride and then reacting the resulting alkali metal derivative with an appropriate ω-chloroalkylamide. The chloroacetanilide derivative used in the process is prepared by a manner as disclosed, for example, in Yakugaku Zasshi (Pharmacological Journal), vol. 99(2), pp. 146 to 154 (1979), which comprises reacting xylidine with monochloroacetyl chloride in a glacial acetic acid to obtain 2, 6-dimethylmonochloroacetanilide in a 78 to 80% yield. However, these processes are not still satisfactory from the viewpoint of yield on an industrial scale.

### SUMMARY OF THE INVENTION

An object of the present invention is to provide a process for preparing halogenoacetamide derivatives which is free from the above-mentioned disadvantages associated with the known process and excellent from the economical standpoint.

Accordingly, the present invention relates to a process for preparing a halogenoacetamide derivative represented by formula (II'): 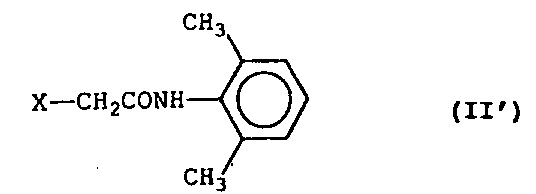 wherein X represents a halogen atom,
comprising reacting an amine represented by formula (III'): 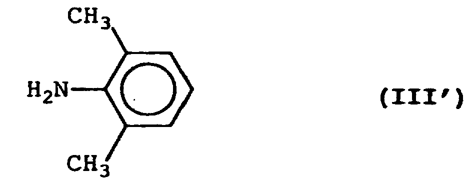 with a halogenoacetyl chloride in the presence of sodium carbonate and in a two-phase reaction system composed of an aqueous layer and an organic solvent layer.

Further, the present invention relates to a process for preparing a 2-(1-pyrrolidinyl)acetamide derivative represented by formula (I'): 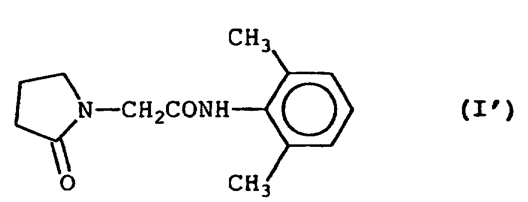 comprising reacting an amine represented by formula (III'): 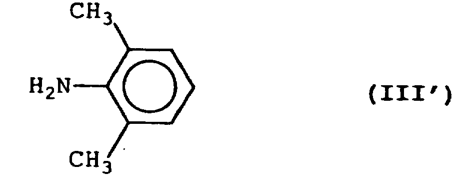 with a halogenoacetyl chloride in the presence of sodium carbonate and in a two-phase reaction system composed of an aqueous layer and an organic solvent layer to obtain a halogenoacetamide derivative represented by formula (II'): 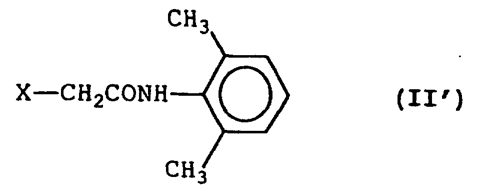 wherein X represents a halogen atom,
and reacting the resulting halogenoacetamide derivative of formula (II') with 2-pyrrolidinone.

According to a particular embodiment, said process comprises reacting 2-pyrrolidinone with a metal alcoholate to obtain a metal salt of 2-pyrrolidinone and reacting the salt with the halogenoacetamide derivative represented by formula (II').

Preferably, the halogenoacetamide derivative is used without isolating it.

### DETAILED DESCRIPTION OF THE INVENTION

2-(1-pyrrolidinyl)acetamide derivatives of formula R²-CH₂-CONH-R¹ are known compounds disclosed in U.S. Patent 4,341,790 and JP-A-56-2960. The substituents which may be on the pyridyl or phenyl group as R¹ include a halogen atom, a trifluoromethyl group, a nitro group, an acetyl group, an alkyl group, an alkoxy group, an alkylmercapto group, an amino group, a sulfonyl group, and an aminoethoxycarbonyl group and the substituent which may be on the 2-oxo-1-pyrrolidinyl group as R² includes a hydroxyl group.

The "alkyl moiety" of the above substituents means a lower alkyl group having from 1 to 5 carbon atoms.

Of said compounds, the most useful is N-(2,6-dimethylphenyl)-2-(2-oxo-1-pyrrolidinyl)-acetamide.

Organic solvents which can be used for formation of an aqueous/organic solvent two phase system where an amine and a halogenoacetyl chloride are reacted are selected from those capable of forming a separate phase when mixed with water or water having dissolved therein an inorganic base or a starting material and preferably those inert to the starting materials. Suitable organic solvents include chloroform, methylene chloride, dichloroethylene, toluene, xylene, ethyl acetate, t-butyl methyl ether, acetone, and acetonitrile. Toluene and xylene are particularly suitable from the viewpoint of recovery and reduced air pollution as well as reaction yield. It is preferable to use the solvent in an amount enough to dissolve the starting materials. However, it is not necessary to use it in an amount enough to dissolve completely the resulting halogenoacetylamide.

The halogenoacetyl chloride which can be used in the present invention is preferably chloroacetyl chloride, which gives 2-chloro-N-(2,6-dimethylphenyl)acetamide.

According to the invention, the aqueous layer is made weakly basic to basic for satisfactory progress of the reaction. To this effect, the base sodium carbonate is preferably added in at least an equivalent amount.

The molar ratio of the halogenoacetyl chloride to the amine is at least 1, and preferably 1.1 to about 1.5. The reaction product (II') can be isolated in a usual manner, for example, (1) by cooling the reaction mixture and collecting the product by filtration, or (2) by separating and concentrating the organic layer and collecting the product by filtration.

Though the intermediate of the formula (II') can be isolated as described above, it is advantageous to use the organic layer in the subsequent step without isolating such intermediate, since there are little by-products. Practically, the organic layer separated is azeotropically distilled under reduced pressure to about 1/2 to 1/3 by volume, thus water content in the organic solvent becomes within an allowable limit (about 0.05% v/v).

Before reacting the resulting halogenoacetamide derivative with 2-pyrrolidinone, it is necessary to treat the 2-pyrrolidinone with a base. Bases which can be used include strong bases, such as potassium hydride, sodium hydride, sodium amide, n-butyl lithium, t-butyl lithium, diethylamino lithium and alcoholates. Of these bases it is preferred to use various alcoholates which are less expensive and less ignitable and to carry out the treatment under heating at about 100 to 150°C for 0.5 to 5 hours while driving the by-produced alcohol out of the system. In particular, sodium methylate and sodium ethylate are preferred because of cheapness and easy removal of by-produced methanol or ethanol. The amount of the base to be used is preferably not more than an equimolar amount with the pyrrolidinone. Use of the base in excess tends to cause side reactions.

The treatment with a base is usually effected in an appropriate solvent which is unreactive with the base. Examples of suitable solvents are toluene, xylene, chloroform, methylene chloride, dichloroethylene, and t-butyl methyl ether. The treatment is preferably conducted in a water-free system because water, if present, may react with the base or may interfere with the treatment.

The halogenoacetamide derivative is added to the pyrrolidinone compound having been treated with a base, and the mixture is allowed to react at a temperature of from 0 to 100°C, and preferably from 40 to 80°C, to obtain the desired pyrrolidinylacetamide derivative. The halogenoacetamide is usually used in an amount not more than an equimolar amount with the base used in the above-mentioned treatment. In carrying out the reaction, it is advantageous from the operation standpoint to use the same solvent as used in the above-mentioned treatment with a base.

The reaction product can easily be isolated by cooling the reaction mixture by addition of water to crystallize the product. This isolation step is also an inventive aspect of the present invention.

The present invention will now be illustrated in greater detail with reference to Examples, but it should be understood that the present invention is not construed as being limited thereto.

### EXAMPLE 1

### Preparation of 2 -Chloro -N-(2,6-dimethylphenyl)acetamide

In 3 ℓ of toluene was dissolved 182 g of 2,6-xylidine, and in 1.5 ℓ of water was dissolved 159 g of sodium carbonate. The two solutions were combined, and 203 g of chloroacetyl chloride was added thereto dropwise over 1.5 hours at an inner temperature of from 20 to 35°C, followed by stirring at that temperature for 1.5 hours. The reaction mixture was cooled with ice, and the precipitated crystals were collected by filtration and dried under reduced pressure to obtain 282 g (95%) of the titled compound.
- Melting point:: 148-148.5°C
- ¹H-NMR (CDCℓ₃):: 2.24 (6H, s), 4.24 (2H, s), 7.16 (3H, s), 7.9 (1H, br s)

### EXAMPLE 2

### Preparation of N-(2,6-Dimethylphenyl)-2-(2-oxo-1-pyrrolidinyl)acetamide

In 300 mℓ of toluene was suspended 9.2 g of 60% sodium hydride in a nitrogen stream, and 21.3 g of 2-pyrrolidinone was slowly added dropwise to the suspension at an inner temperature controlled at 40°C or lower. After stirring the mixture for 2 hours, 19.7 g of 2-chloro-N-(2,6-dimethylphenyl)acetamide was added thereto, and the mixture was allowed to react at 60 to 70°C for 2 hours. To the reaction mixture was added 50 mℓ of water, followed by allowing to cool with stirring. The precipitated crystals were collected by filtration and dried under reduced pressure to obtain 22.2 g (90%) of the titled compound.
- Melting point:: 153-153.5°C
- ¹H-NMR (CDCℓ₃):: 1.9-2.6 (4H, m), 2.18 (6H, s), 3.57 (2H, t, J=6.8Hz), 4.07 (2H, s), 7.06 (3H, s), 7.9 (1H, br s)

### EXAMPLE 3

### Preparation of N-(2,6-Dimethylphenyl)-2-(2-oxo-1-pyrrolidinyl)acetamide

In 30 mℓ of t-butyl methyl ether was suspended 897 mg of sodium amide in a nitrogen stream, and 2.13 g of 2-pyrrolidinone was slowly added thereto dropwise, followed by refluxing for 2 hours. After allowing to cool, 1.97 g of 2-chloro-N-(2,6-dimethylphenyl)acetamide was added thereto, followed by refluxing for 2 hours. To the reaction mixture was added 50 mℓ of water, and the mixture was allowed to cool with stirring. The thus formed crystals were collected by filtration and dried under reduced pressure to obtain 2.19 g (89%) of the titled compound.
Melting point: 151-152.5°C

### EXAMPLE 4

### Preparation of N-(2,6-Dimethylphenyl)-2-(2-oxo-1-pyrrolidinyl)acetamide

In 300 mℓ of toluene was suspended 9.0 g of sodium amide in a nitrogen stream, and 21.3 g of 2-pyrrolidinone was slowly added thereto dropwise. After stirring the mixture at 60 to 70°C for 2 hours, 19.7 g of 2-chloro-N-(2,6-dimethylphenyl)acetamide was added thereto, followed by allowing to react at 60 to 70°C for 2 hours. To the reaction mixture was added 50 mℓ of water, and the mixture was allowed to cool while stirring. The precipitated crystals were recovered by filtration and dried under reduced pressure to obtain 22.4 g (91%) of the titled compound.
Melting point: 152-152.5°C

### EXAMPLE 5

### Preparation of N-(2,6-Dimethylphenyl)-2-(2-oxo-1-pyrrolidinyl)acetamide

In 5 ℓ of toluene was suspended 203 g of sodium methylate, and 511 g of 2-pyrrolidinone was added thereto. The mixture was slowly heated up to 100 to 110°C, at which it was allowed to react for 3 hours. During the reaction, about 300 mℓ of toluene was distilled off. After allowing to cool, 296 g of 2-chloro-N-(2,6-dimethylphenyl)acetamide was added thereto to react at 60 to 70°C for 2 hours. To the reaction mixture was added 300 mℓ of water, followed by allowing to cool with stirring. The crystals formed were collected by filtration and dried under reduced pressure to obtain 332 g (90%) of the titled compound.
Melting point: 152.5-153°C

### EXAMPLE 6

### Preparation of N-(2,6-Dimethylphenyl)-2-(2-oxo-1-Pyrrolidinyl)acetamide

In 5 ℓ of toluene was dissolved 182 g of 2,6-xylidine, and in 1.5 ℓ of water was dissolved 159 g of sodium carbonate. The two solutions were combined, and 203 g of chloroacetyl chloride was added thereto dropwise over 1.5 hours at an inner temperature of from 20 to 35°C, followed by stirring at that temperature for 1.5 hours. The reaction mixture was heated to about 70°C to dissolve the precipitated crystals and then separated into two layers. The organic layer was concentrated under reduced pressure to about 500 mℓ. The resulting suspension of 2-chloro-N-(2,6-dimethylphenyl)acetamide was allowed to cool.

Separately, 203 g of sodium methylate was suspended in 4 ℓ of toluene, and 511 g of 2-pyrrolidinone was added thereto. The mixture was slowly heated up to 100 to 110°C, at which it was allowed to react for 3 hours. During the reaction, about 300 mℓ of toluene was distilled off. After allowing to cool, the above prepared suspension of 2-chloro-N-(2,6-dimethylphenyl)acetamide was added thereto to react at 60 to 70°C for 2 hours. To the reaction mixture was added 300 mℓ of water, followed by allowing to cool with stirring. The crystals formed were collected by filtration and dried under reduced pressure to obtain 336 g (91%) of the titled compound.
Melting point: 152-153°C

While the invention has been described in detail and with reference to specific examples thereof, it will be apparent to one skilled in the art that various changes and modifications can be made therein without departing from the spirit and scope thereof.

## Claims

1. A process for preparing a halogenoacetamide derivative represented by formula (II'): 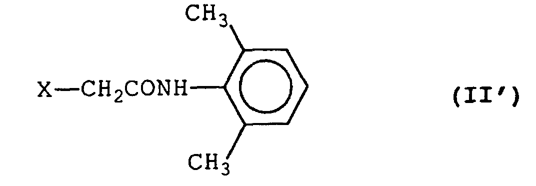 wherein X represents a halogen atom,
comprising reacting an amine represented by formula (III'): 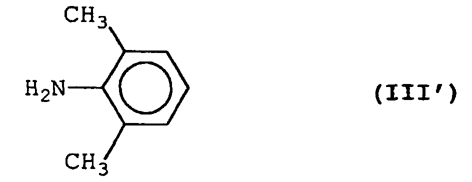 with a halogenoacetyl chloride in the presence of sodium carbonate and in a two-phase reaction system composed of an aqueous layer and an organic solvent layer.

2. A process as claimed in Claim 1, wherein said halogenoacetyl chloride is chloroacetyl chloride, and said halogenoacetamide derivative is 2-chloro-N-(2,6-dimethylphenyl)acetamide.

3. A process for preparing a 2-(-1-pyrrolidinyl)acetamide derivative represented by formula (I'): 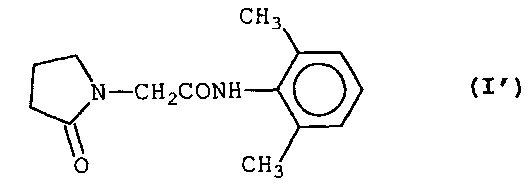 comprising reacting an amine represented by formula (III'): 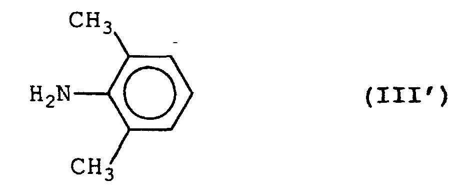 with a halogenoacetyl chloride in the presence of sodium carbonate and in a two-phase reaction system composed of an aqueous layer and an organic solvent layer to obtain a halogenoacetamide derivative represented by formula (II'): 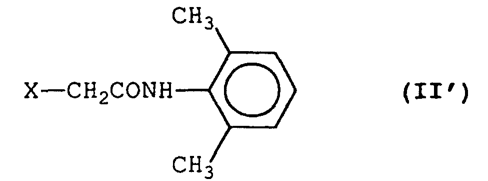 wherein X represents a halogen atom,
and reacting the resulting halogenoacetamide derivative of formula (II') with 2-pyrrolidinone.

4. The process of Claim 3 comprising reacting 2-pyrrolidinone with a metal alcoholate to obtain a metal salt of 2-pyrrolidinone and reacting the salt with the halogenoacetamide derivative represented by formula (II').

5. The process of Claim 4, wherein said metal alcoholate is sodium methylate or sodium ethylate.

6. The process of any of Claims 3 to 5, wherein the halogenoacetamide derivative is used without isolating it.

## Patentansprüche

1. Verfahren zur Herstellung eines Halogenacetamid-Derivats der Formel (II'): 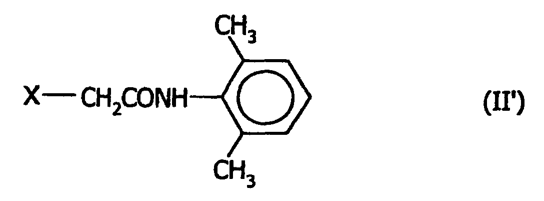 worin X ein Halogenatom bedeutet,
durch Umsetzung eines Amins der Formel (III'): 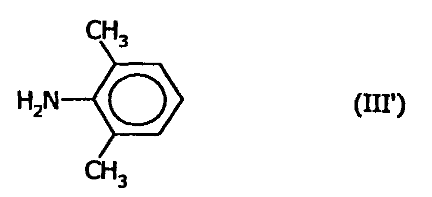 mit einem Halogenacetylchlorid in Gegenwart von Natriumcarbonat und einem aus einer wässrigen Schicht und einer organischen Lösungsmittelschicht gebildeten, zweiphasigen Reaktionssystem.

2. Verfahren nach Anspruch 1, wobei das Halogenacetylchlorid Chloracetylchlorid ist, und das Halogenacetamid-Derivat 2-Chlor-N-(2,6-Dimethylphenyl)acetamid ist.

3. Verfahren zur Herstellung eines 2-(1-Pyrrolidinyl)-acetamid-Derivats der Formel (I'): 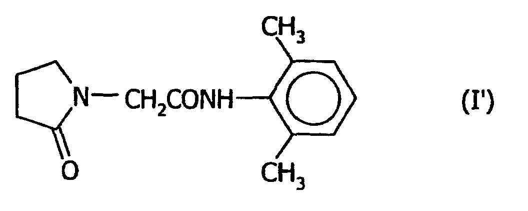 durch Umsetzung eines Amins der Formel (III'): 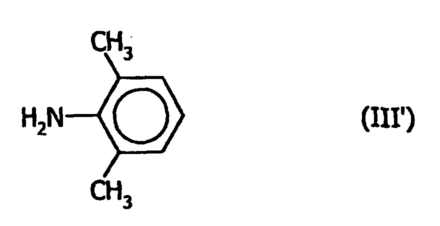 mit einem Halogenacetylchlorid in Gegenwart von Natriumcarbonat und einem aus einer wässrigen Schicht und einer organischen Lösungsmittelschicht gebildeten, zweiphasigen Reaktionssystem zu einem Halogenacetamid-Derivat der Formel (II'): 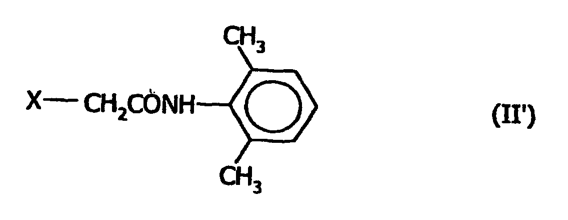 worin X ein Halogenatom bedeutet,
und durch Umsetzung des resultierenden Halogenacetamid-Derivats der Formel (II') mit 2-Pyrrolidinon.

4. Verfahren nach Anspruch 3, wobei man 2-Pyrrolidinon mit einem Metallalkoholat zu einem Metallsalz von 2-Pyrrolidinon umsetzt und das Salz mit dem Halogenacetamid-Derivat der Formel (II') umsetzt.

5. Verfahren nach Anspruch 4, wobei das Metallalkoholat Natriummethylat oder Natriumethylat ist.

6. Verfahren nach einem der Ansprüche 3 bis 5, wobei das Halogenacetamid-Derivat verwendet wird, ohne es zu isolieren.

## Revendications

1. Procédé pour la préparation d'un dérivé d'halogénoacétamide représenté par la formule (II') : 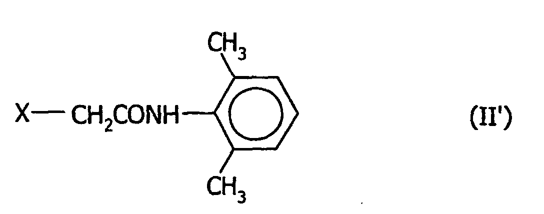 où X représente un atome d'halogène,
comprenant la réaction d'une amine représentée par la formule (III') : 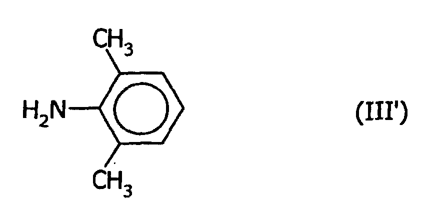 avec un chlorure d'halogénoacétyle en présence de carbonate de sodium et dans un système réactionnel à deux phases constitué d'une couche aqueuse et d'une couche de solvant organique.

2. Procédé selon la revendication 1, dans lequel ledit chlorure d'halogénoacétyle est le chlorure de chloroacétyle et ledit dérivé d'halogénoacétamide est le 2-chloro-N-(2,6-diméthylphényl)acétamide.

3. Procédé pour la préparation d'un dérivé de 2-(1-pyrroiidinyl)acétamide représenté par la formule (I') : 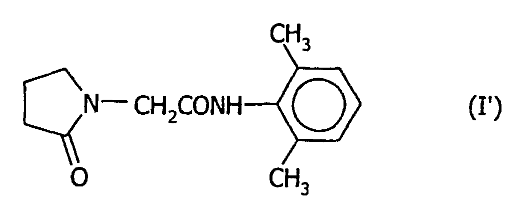 comprenant la réaction d'une amine représentée par la formule (III') : 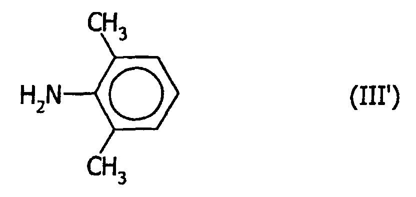 avec un chlorure d'halogénoacétyle en présence de carbonate de sodium et dans un système réactionnel à deux phases constitué d'une couche aqueuse et d'une couche de solvant organique pour obtenir un dérivé d'halogénoacétamide représenté par la formule (II') : 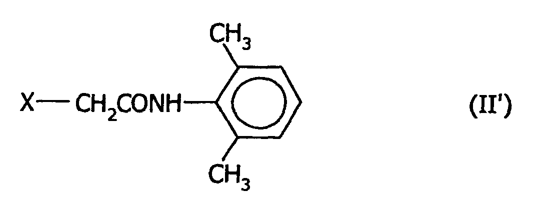 où X représente un atome d'halogène,
et la réaction du dérivé d'halogénoacétamide résultant de la formule (II') avec de la 2-pyrrolidinone.

4. Procédé selon la revendication 3, comprenant la réaction de 2-pyrrolidinone avec un alcoolate de métal pour obtenir un sel métallique de 2-pyrrolidinone et la réaction du sel avec le dérivé d'halogénoacétamide représenté par la formule (II').

5. Procédé selon la revendication 4, dans lequel ledit alcoolate de métal est le méthylate de sodium ou l'éthylate de sodium.

6. Procédé selon l'une quelconque des revendications 3 à 5, dans lequel le dérivé d'halogénoacétamide est utilisé sans l'isoler.
